# EUROPEAN PATENT SPECIFICATION

(11) **EP 1 991 248 B1**
(45) Date of publication and mention of the grant of the patent: **11.04.2012**
(21) Application number: 07751499.0
(22) Date of filing: 22.02.2007
(51) Int. Cl.: A61K 31/195, A61K 47/48

(54) **ANTIDEPRESSANT PRODRUGS**
ANTIDEPRESSIVUM-PROPHARMAKA
PROMÉDICAMENTS ANTIDÉPRESSEURS

(30) Priority: 24.02.2006 US 776216 P
(43) Date of publication of application: 19.11.2008
(73) Proprietor: Shire LLC, Florence, KY 41042 (US)
(72) Inventor: MICKLE, Travis, Coralville, Iowa 52241 (US); SEVERS, Wendy Hirschelman, Blacksburg, Virginia 24060 (US)
(74) Representative: Harrison Goddard Foote
(86) International application number: PCT/US2007/004743
(87) International publication number: WO 2007/100668

(56) References cited:
- WO-A-2005/032474
- US-A- 3 875 137
- US-A1- 2005 176 646
- US-B1- 6 569 850

## Description

### FIELD OF THE INVENTION

This disclosure relates to antidepressant prodrugs comprising an antidepressant covalently bound to a chemical moiety, particularly one or more amino acids. This disclosure also relates to pharmaceutical compositions comprising an antidepressant prodrug, and to methods of preparing and using the same.

### BACKGROUND OF THE INVENTION

Antidepressants are a class of psychotherapeutic drugs that are prescribed to treat a variety of psychiatric and medical disorders. According to the U.S. Substance Abuse and Mental Health Services Administration's (SAMHSA) 2004 National Survey on Drug Use and Health, an estimated 8% of adults experienced at least one major depressive episode during the past year. While antidepressants can be used as effective therapy, they also present a risk of overdose, especially for suicidal patients who may have both access and intent to consume high doses. Townsend, E. et al. "Substances used in deliberate self-poisoning 1985-1997: trends and associations with age, gender, repetition and suicide intent" Soc. Psychiatry Psychiatr. Epidemiol. 36: 228-34 (2001). According to the Drug Abuse Warning Network (DAWN), of the drugs mentioned in drug-related emergency department visits in 2002, antidepressants accounted for 5%.

Older classes of antidepressants, including monoamine oxidase inhibitors (MAOIs) and especially tricyclic antidepressants, can cause adverse cardiovascular effects and are highly toxic in overdose, especially when combined with other drugs. Newer antidepressants, including selective serotonin reuptake inhibitors (SSRIs), were developed in part to reduce the risk of toxicity in overdose. Sarko J. "Antidepressants, Old and New: A Review of Their Adverse Effects and Toxicity in Overdose." Emerg. Med. Clin. North Am. 18(4): 637-54, 637, 639, 646 (2000); Glauser, J. "Tricyclic Antidepressant Poisoning" Clev. Cl. J. of Med. 67(10): 704-19, 709 (2000). While SSRIs have a reduced risk of cardiovascular side effects compared to tricyclics, SSRIs do have adverse side effects, most commonly nausea, vomiting, and diarrhea. Sarko at 638.

US 3,875,137 discloses peptides containing the residues of methionine and aspartic acid that are prepared by a standard coupling technique and display valuable pharmacological, e.g. gastrin-inhibitory, properties.

WO 2005/032474 discloses pharmaceutical compositions comprised of a chemical moiety attached to an active agent in a manner that substantially decreases the potential of the active agent to cause overdose or to be abused. When delivered at the proper dosage, the pharmaceutical composition provides therapeutic activity similar to that of the parent active agent.

US 2005/0176646 also discloses pharmaceutical compositions comprised of a chemical moiety attached to an active agent in a manner that substantially decreases the potential of the active agent to cause overdose or to be abused. When delivered at the proper dosage, the pharmaceutical composition provides therapeutic activity similar to that of the parent active agent.

US 6,569,850 discloses a method for treating multiple sclerosis or encephalomyelitis by administering to a patient a combination of a tricyclic antidepressant drug and tyrosine or phenylalanine or both.

In this invention, one or more amino acids is conjugated to tranylcypromine. Among the advantages, the resulting prodrug may be resistant to overdose due to a natural gating mechanism at the site of hydrolysis that limits the release of free drug. The prodrug may also reduce side effects such as diarrhoea, upset stomach, vomiting, and weight loss.

### BRIEF DESCRIPTION OF DRAWINGS

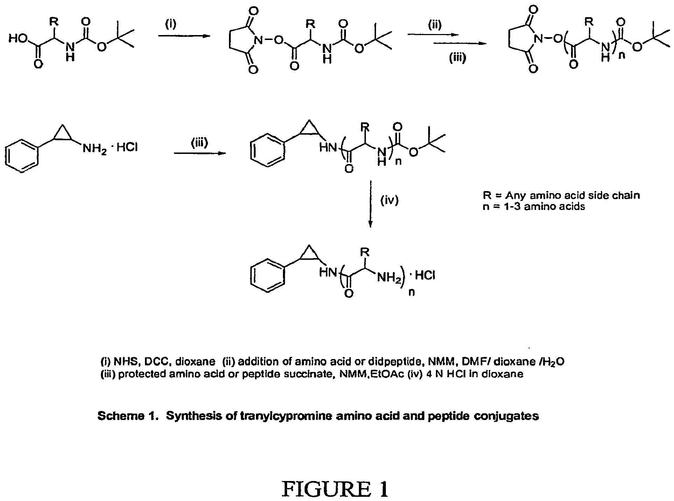

FIG. 1 depicts a process for preparing tranylcypromine prodrugs.

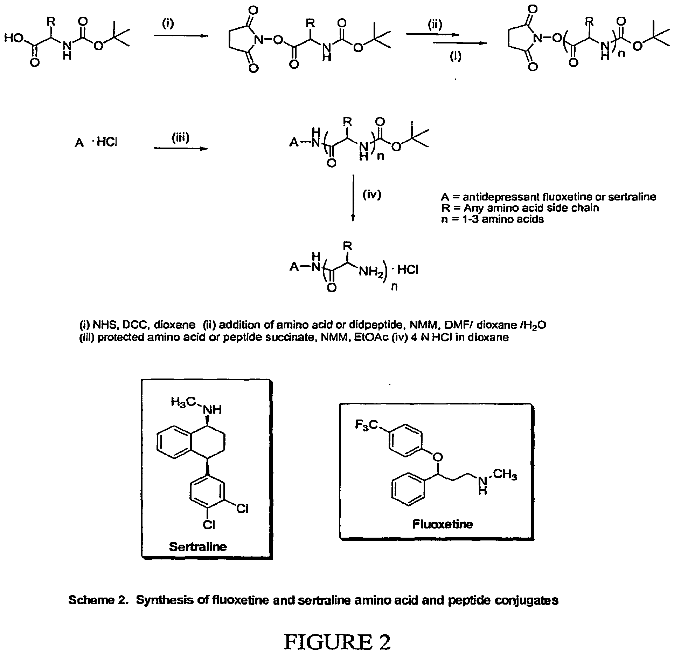

FIG. 2 depicts a process for preparing fluoxetine and sertraline prodrugs. These prodrugs do not fall within the scope of the invention as claimed, but the figures are retained as useful information to the reader.

### DETAILED DESCRIPTION OF THE INVENTION

According to a first aspect, the present invention provides a composition, or salt thereof, comprising tranylcypromine covalently bound to either: a single amino acid; a dipeptide in which the two amino acids are the same; or a tripeptide in which the three amino acids are the same and wherein the amino acid is a naturally occurring amino acid or a synthetic amino acid selected from the group comprising:
aminohexanoic acid, biphenylalanine, cyclohexylalanine, cyclohexylglycine, diethyglycine, dipropylglycine, 2,3-diaminoproprionic acid, homophenylalanine, homoserine, homotyrosine, naphthylalanine, norleucine, ornithine, phenylalanine (4-fluoro), phenylalanine (2,3,4,5,6-pentafluoro), phenylalanine (4-nitro), phenylglycine, pipecolic acid, sarcosine, tetrahydroisoquinoline-3-carboxcylic acid, tert-leucine, and an amino acid with a C₁-C₁₇ alkyl side chain.

According to a second aspect, the present invention provides a pharmaceutical composition comprising a composition of the present invention and at least one pharmaceutical additive.
The structure of tranylcypromine is as follows:

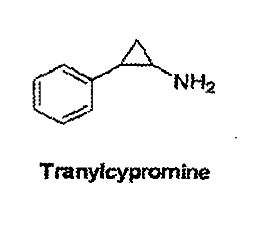

The antidepressant can have any stereogenic configuration, including both dextro- and levo- isomers.

The prodrug can be in a salt or ester form. Exemplary salts are known in the pharmaceutical art and include, for example, mesylate, hydrochloride, and sulfate salts.

Tranylcypromine is covalently bound (conjugated) to a peptide comprising one or more amino acids. An amino acid can be bound to the drug via the N-terminus, the C-terminus, or a side chain of the amino acid. The drug can be bound to the peptide via its amino group. The drug can be covalently bound to the peptide either directly, or indirectly through a linker. Covalent attachment can be, e.g., an ester or carbonate bond.

As used herein, "peptide" includes single amino acids, dipeptides, and tripeptides.

Each amino acid can be any of the naturally occurring amino acids: alanine (Ala or A), arginine (Arg or R), asparagine (Asn or N), aspartic acid (Asp or D), cysteine (Cys or C), glycine (Gly or G), glutamic acid (Glu or E), glutamine (Gln or Q)5 histidine (His or H), isoleucine (He or I), leucine (Leu or L), lysine (Lys or K), methionine (Met or M)5 proline (Pro or P), phenylalanine (Phe or F), serine (Ser or S), tryptophan (Trp or W), threonine (Thr or T), tyrosine (Tyr or Y), and valine (Val or V). Each amino acid can be an L- or D- enantiomer; L-enantiomers are preferred. In a preferred embodiment, the prodrug comprises only naturally occurring amino acids and/or only L-amino acids.

Each amino acid can be an unnatural, non-standard, or synthetic amino acid, such as aminohexanoic acid, biphenylalanine, cyclohexylalanine, cyclohexylglycine, diethylglycine, dipropylglycine, 2,3 diaminoproprionic acid, homophenylalanine, homoserine, homotyrosine, naphthylalanine, norleucine, ornithine, phenylalanine (4-fluoro), phenylalanine(2,3, 4,5,6 pentafluoro), phenylalanine(4-nitro), phenylglycine, pipecolic acid, sarcosine, tetrahydroisoquinoline-3-carboxylic acid, and tert-leucine. Preferably, synthetic amino acids with alkyl side chains are selected from C₁-C₁₇ alkyls, preferably C₁-C₆ alkyls.

In one embodiment, the prodrug comprises a dipeptide or a tripeptide wherein each of the amino acids is the same, such as, Gly-Gly- Gly (Gly₃). In addition to the first amino acid bound to the antidepressant, one or more additional amino acids can be bound to the first amino acid at a terminus or side chain.

The peptide can comprise a homopolymer of naturally- occurring or synthetic amino acids, such as a homopolymer of glutamic acid, aspartic acid, serine, lysine, cysteine, threonine, asparagine, arginine, tyrosine, or glutamine.

In one embodiment, an amino acid has one or more free C-terminal, N-terminal, and/or side chain group other than the point of attachment to the drug. The amino acid can be in such a free state, or it can be an ester or salt thereof.

Exemplary peptides for the antidepressant prodrugs of the present invention include Ala, Arg, Asn, Asp, Gly, Glu, His, He, Leu, Lys, Met, Pro, Phe, Ser, Trp, Thr, Tyr, Phe₂, Gly₂, Glu₂, Pro₂, Lys₂, Asp₂, and Gly₃.

Exemplary methods of attaching one or more amino acids to a drug are described in the Examples below. Additional methods related to peptide drug conjugates are described in U.S. Pat. No. 6,716,452, WO 03/072735, and WO 03/101476. Synthesis of amino acid and peptide conjugates can be verified using nuclear magnetic resonance (NMR), high resolution mass spectroscopy, and/or other methods known in the art.

The antidepressant prodrugs described above may exhibit one or more of the following advantages over the unbound antidepressant. The prodrug exhibits similar pharmacological activity to the unbound drug at therapeutic doses. The prodrug prevents abuse and/or overdose by exhibiting a reduced pharmacological activity when administered at higher than therapeutic doses (suprapharmacological doses), e.g., higher than the prescribed dose. The prodrug exhibits reduced side effects compared to the unbound drug.

The phrase "similar pharmacological activity" means that two compounds exhibit curves that have substantially the same pharmacological parameters (AUC, Cₘₐₓ, Tₘₐₓ, Cₘᵢₙ, and/or t_{1/2}), preferably within about 30% of each other, more preferably within about 25%, 20%, 10%, 5%, 2%, 1 %, or increments therein of each other. In one embodiment, at least one pharmacological parameter is within 80% to 125%, 80% to 120%, 85% to 125%, 90% to 110%, or increments therein, of each other.

Throughout this application, the term "increments therein" is used to define a range in varying degrees of precision, e.g., to the nearest 10, 1, 0.1, 0.01, etc. The increment can be rounded to any measurable degree of precision. For example, the range 1 to 100 or increments therein includes ranges such as 20 to 80, 5 to 50, 0.4 to 98, and 0.04 to 98.05.

Use of the term "reduced" or "increased" includes at least a 10% change with greater percentage changes being preferred. For instance, the change can be greater than 25%, 35%, 45%, 55%, 65%, 75%, 85%, 95%, 96%, 97%, 98%, 99%, or increments therein.

Without being bound by theory, it is believed that the prodrug is resistant to abuse and/or overdose due to a natural gating mechanism at the site of hydrolysis, namely the gastrointestinal tract. This gating mechanism is thought to allow the release of therapeutic amounts of antidepressant from the antidepressant prodrug, but limit the release of higher amounts of antidepressant. The prodrug may also have a different metabolism and/or selective delivery resulting in fewer adverse side effects.

In one embodiment, the toxicity of the prodrug is substantially lower than that of the unbound drug. For example, in a preferred embodiment, the acute toxicity is 1- fold, 2-fold, 3-fold, 4-fold, 5-fold, 6-fold, 7-fold, 8-fold, 9-fold, 10-fold, or increments therein less lethal than administration of unbound drug. Preferably, the prodrug provides a serum release curve which does not increase above the drug's toxicity level when administered at higher than therapeutic doses. The prodrug may exhibit a reduced rate of drug absorption and/or an increased rate of clearance compared to the unbound drug. The prodrug may also exhibit a steady-state serum release curve. Preferably, the prodrug provides bioavailability but prevents Cₘₐₓ spiking or increased blood serum concentrations.

In another embodiment, the invention provides a composition for use in the treatment of major depressive disorder, obsessive-compulsive disorder (OCD), bulimia nervosa, panic disorder with and without agoraphobia, posttraumatic stress disorder (PTSD), premenstrual dysphoric disorder (PMDD), social anxiety disorder (social phobia), major depressive episode without melancholia, generalized anxiety disorder, depression with and without prominent anxiety, endogenous depression, other psychiatric disorders, and nicotine withdrawal. Preferred indications include major depressive disorder, OCD, bulimia nervosa, panic disorder with and without agoraphobia, PTSD, PMDD, social anxiety disorder, and major depressive episode without melancholia. Treating includes preventing, ameliorating, and/or eliminating the symptoms of a disease.

The compositions of the invention can administered in combination therapies comprising administering one or more therapeutic agents in addition to an antidepressant prodrug. The additional therapeutic agent can be, for example, a benzodiazepine, neuroleptic, lithium, thyroid supplement, or an additional antidepressant or antidepressant prodrug. The therapeutic agents can be formulated into a single dosage form, or they can be formulated together or separately among multiple dosage forms. The therapeutic agents can be administered simultaneously or sequentially in any order. The methods for treatment also include combining the administration of an antidepressant prodrug with non-drug therapies such as cognitive therapy, electroconvulsive therapy, light therapy, etc.

The prodrug may exhibit delayed and/or sustained release characteristics. Delayed release prevents rapid onset of pharmacological effects, and sustained release is a desirable feature for particular dosing regimens, e.g., once a day regimens. The prodrug may achieve the release profile independently. Alternatively, the prodrug can be pharmaceutically formulated to enhance or achieve such a release profile. It may be desirable to reduce the amount of time until onset of pharmacological effect, e.g., by admixture with an immediate release product.

The prodrug may exhibit increased stability as compared to the unbound drug. The prodrug may achieve increased stability independently. Alternatively, the prodrug can be pharmaceutically formulated to enhance or achieve increased stability.

Because the prodrug may independently achieve a desirable release profile and/or stability, these desirable characteristics need not depend on a dissolution process and/or the water solubility of the pharmaceutical composition. This provides a further advantage of reliable dosing and batch to batch reproducibility.

In another embodiment, the prodrug exhibits increased solubility in aqueous or non-aqueous solutions as compared to the unbound drug. Increased solubility in aqueous solutions, such as those found in the intestinal tract, provide improved bioavailability of the drug. Increased solubility in organic solvents such as isopropanol and acetone allows even dispersion of the drug in polymer formulations that require certain organic solvents.

In another embodiment, the invention provides pharmaceutical compositions comprising an antidepressant prodrug as defined in the claims. In addition to a prodrug, the pharmaceutical compositions of the invention can further comprise one or more pharmaceutical additives. Pharmaceutical additives include a wide range of materials including, but not limited to, diluents and bulking substances, binders and adhesives, lubricants, glidants, plasticizers, disintegrants, carriers and solvents, buffers, colorants, flavorings, sweeteners, preservatives and stabilizers, and other pharmaceutical additives known in the art.

Diluents increase the bulk of a dosage form and may make the dosage form easier to handle. Exemplary diluents include, but are not limited to, lactose, dextrose, saccharose, cellulose, starch, and calcium phosphate for solid dosage forms, e.g., tablets and capsules; olive oil and ethyl oleate for soft capsules; water and vegetable oil for liquid dosage forms, e.g., suspensions and emulsions. Additional suitable diluents include, but are not limited to, sucrose, dextrates, dextrin, maltodextrin, microcrystalline cellulose (e.g., Avicel®), microfine cellulose, powdered cellulose, pregelatinized starch (e.g., Starch 1500®), calcium phosphate dihydrate, soy polysaccharide (e.g., Emcosoy®), gelatin, silicon dioxide, calcium sulfate, calcium carbonate, magnesium carbonate, magnesium oxide, sorbitol, mannitol, kaolin, polymethacrylates (e.g., Eudragit®), potassium chloride, sodium chloride, and talc.

In embodiments where the pharmaceutical composition is compacted into a solid dosage form, e.g., a tablet, a binder can help the ingredients hold together. Binders include, but are not limited to, sugars such as sucrose, lactose, and glucose; corn syrup; soy polysaccharide, gelatin; povidone (e.g., Kollidon®, Plasdone®); Pullulan; cellulose derivatives such as microcrystalline cellulose, hydroxypropylmethyl cellulose (e.g., Methocel®), hydroxypropyl cellulose (e.g., Klucel®), ethylcellulose, hydroxyethyl cellulose, carboxymethylcellulose sodium, and methylcellulose; acrylic and methacrylic acid co-polymers; carbomer (e.g., Carbopol®); polyvinylpolypyrrolidine, polyethylene glycol (Carbowax®); pharmaceutical glaze; alginates such as alginic acid and sodium alginate; gums such as acacia, guar gum, and arabic gums; tragacanth; dextrin and maltodextrin; milk derivatives such as whey; starches such as pregelatinized starch and starch paste; hydrogenated vegetable oil; and magnesium aluminum silicate.

For tablet dosage forms, the pharmaceutical composition is subjected to pressure from a punch and dye. Among other purposes, a lubricant can help prevent the composition from sticking to the punch and dye surfaces. A lubricant can also be used in the coating of a coated dosage form. Lubricants include, but are not limited to, magnesium stearate, calcium stearate, zinc stearate, powdered stearic acid, glyceryl monostearate, glyceryl palmitostearate, glyceryl behenate, silica, magnesium silicate, colloidal silicon dioxide, titanium dioxide, sodium benzoate, sodium lauryl sulfate, sodium stearyl fumarate, hydrogenated vegetable oil, talc, polyethylene glycol, and mineral oil.

Glidants can improve the flowability of non-compacted solid dosage forms and can improve the accuracy of dosing. Glidants include, but are not limited to, colloidal silicon dioxide, fumed silicon dioxide, silica gel, talc, magnesium trisilicate, magnesium or calcium stearate, powdered cellulose, starch, and tribasic calcium phosphate.

Plasticizers include both hydrophobic and hydrophilic plasticizers such as, but not limited to, diethyl phthalate, butyl phthalate, diethyl sebacate, dibutyl sebacate, triethyl citrate, acetyltriethyl citrate, acetyltributyl citrate, cronotic acid, propylene glycol, castor oil, triacetin, polyethylene glycol, propylene glycol, glycerin, and sorbitol. Plasticizers are particularly useful for pharmaceutical compositions containing a polymer and in soft capsules and film-coated tablets.

Disintegrants can increase the dissolution rate of a pharmaceutical composition. Disintegrants include, but are not limited to, alginates such as alginic acid and sodium alginate, carboxymethylcellulose calcium, carboxymethylcellulose sodium (e.g., Ac-Di-Sol®, Primellose®), colloidal silicon dioxide, croscarmellose sodium, crospovidone (e.g., Kollidon®, Polyplasdone®), polyvinylpolypyrrolidine (Plasone-XL®), guar gum, magnesium aluminum silicate, methyl cellulose, microcrystalline cellulose, polacrilin potassium, powdered cellulose, starch, pregelatinized starch, sodium starch glycolate (e.g., Explotab®, Primogel®).

In embodiments where the pharmaceutical composition is formulated for a liquid dosage form, the pharmaceutical composition may include one or more solvents. Suitable solvents include, but are not limited to, water; alcohols such as ethanol and isopropyl alcohol; methylene chloride; vegetable oil; polyethylene glycol; propylene glycol; and glycerin. Liquid dosage forms such as syrups, emulsions, or suspensions can contain a carrier, for example, a natural gum, agar, sodium alginate, pectin, methylcellulose, carboxymethylcellulose, saccharose, saccharose with glycerol, mannitol, sorbitol, and polyvinyl alcohol.

The pharmaceutical composition can comprise a buffer. Buffers include, but are not limited to, lactic acid, citric acid, acetic acid, sodium lactate, sodium citrate, and sodium acetate.

Any pharmaceutically acceptable colorant can be used to improve appearance or to help identify the pharmaceutical composition. See 21 C.F.R., Part 74. Exemplary colorants include D&C Red No. 7, FD&C Red No. 40, D&C Yellow No. 6, D&C Yellow No. 10, iron oxide, FD&C Blue No. 1, FD&C Blue No. 2, and edible inks.

Flavorings improve palatability and may be particularly useful for chewable tablet or liquid dosage forms. Flavorings include, but are not limited to maltol, vanillin, ethyl vanillin, menthol, citric acid, fumaric acid, ethyl maltol, and tartaric acid. Sweeteners include, but are not limited to, sorbitol, saccharin, sodium saccharin, sucrose, aspartame, fructose, mannitol, and invert sugar.

The pharmaceutical compositions of the invention can also include one or more preservatives and/or stabilizers to improve storagability. These include, but are not limited to, alcohol, sodium benzoate, butylated hydroxy toluene, butylated hydroxyanisole, and ethylenediamine tetraacetic acid.

Other pharmaceutical additives include microencapsulating agents; gelling agents such as colloidal clays; thickening agents such as gum tragacanth and sodium alginate; wetting agents such as lecithin, polysorbates, and laurylsulphates; humectants; antioxidants such as vitamin E, caronene, and BHT; adsorbents; effervescing agents; emulsifying agents, viscosity enhancing agents; surface active agents such as sodium lauryl sulfate, dioctyl sodium sulfosuccinate, triethanolamine, polyoxyethylene sorbitan, poloxalkol, and quaternary ammonium salts; and other miscellaneous excipients such as polysorbate 80, xylose, galactose, maltose, xylitol, and chloride, sulfate and phosphate salts of potassium, sodium, and magnesium.

The pharmaceutical composition can include a hydrophilic polymer to enhance or achieve a sustained release profile. Suitable hydrophilic polymers include, but are not limited to, natural or partially or totally synthetic hydrophilic gums such as acacia, gum tragacanth, locust bean gum, guar gum, and karaya gum; cellulose derivatives such as methyl cellulose, hydroxymethyl cellulose, hydroxypropylmethyl cellulose, hydroxypropyl cellulose, hydroxyethyl cellulose, and carboxymethyl cellulose; hypromellose; proteinaceous substances such as agar, pectin, carrageen, and alginates; hydrophilic polymers such as carboxypolymethylene; gelatin; casein; zein; bentonite; magnesium aluminum silicate; polysaccharides; modified starch derivatives; and other hydrophilic polymers known in the art. One of ordinary skill in the art would recognize a variety of structures, such as bead constructions and coatings, useful for achieving particular release profiles.

The pharmaceutical compositions can be manufactured according to any method known to those of ordinary skill in the art of pharmaceutical manufacture such as, for example, wet granulation, dry granulation, encapsulation, direct compression, slugging, etc. For instance, a pharmaceutical composition can be prepared by mixing the prodrug with one or more pharmaceutical additives with an aliquot of liquid, preferably water, to form a wet granulation. The wet granulation can be dried to obtain granules. The resulting granulation can be milled, screened, and/or blended with various pharmaceutical additives. After granulation, the pharmaceutical composition can be encapsulated, e.g., in a gelatin capsule. Alternatively, the pharmaceutical composition can be tableted, e.g., compressed and optionally coated with a protective coating that dissolves or disperses in gastric juices.

The pharmaceutical compositions of the invention can be administered by a variety of dosage forms. Any biologically-acceptable dosage form known in the art, and combinations thereof, are contemplated. Examples of preferred dosage forms include, but are not limited to: tablets including chewable tablets, film-coated tablets, quick dissolve tablets, effervescent tablets, multi-layer tablets, and bi-layer tablets; caplets; powders including reconstitutable powders; granules; dispersible granules; particles; microparticles; capsules including soft and hard gelatin capsules; lozenges; chewable lozenges; cachets; beads; liquids; solutions or suspensions in an aqueous or non-aqueous liquid; emulsions such as an oil-in-water liquid emulsion or a water-in-oil liquid emulsion; elixirs; and syrups.

The pharmaceutical compositions can be administered by a variety of routes including oral, buccal, parenteral (such as subcutaneous, intramuscular, and intravenous), intranasal, rectal, and topical administration. Oral administration is preferred as it may permit the maximum release of the drug, provide sustained release of the drug, and/or maintain abuse resistance.

The extent of absorption for orally administered drugs is critical in determining the serum level or the concentration of the drug in the systemic circulation. Once in the bloodstream, the drug molecule may experience a variety of fates including binding to serum proteins, distribution to its locus of action (the desired fate) as well as tissue reservoirs, biotransformation, or metabolism, and ultimately, excretion. These fates are preceded by the initial process of absorption. Although the oral route may be the safest and most convenient route, it does impart a relatively high degree of variability. One of the reasons that the oral route is safe is because drugs in the gastrointestinal (GI) tract may be metabolized by enzymes (from the intestinal flora, the mucosa, and the liver) prior to their arrival into the general circulation. The metabolism of drugs occurring between absorption and systemic circulation is referred to as "the first pass effect" or the "hepatic pass", specifically referring to liver detoxification.

Dosing regimens can be optimized by measuring serum levels after a set dose and calculating relevant parameters, but this is not done routinely. The optimization of dosing regimens is more commonly determined by the more practical method of measuring a therapeutic drug effect and adjusting dosage until the desired effect is achieved. In cases where the therapeutic effect is more subjective, such as many of the drugs commonly used to treat psychiatric disorders, doses may be adjusted to avoid adverse effects such as nausea and dizziness. Since therapeutic drug monitoring is often difficult outside a hospital, any help in decreasing the variation between patients will be of practical significance in the determination of dosing instructions.

Oral dosage forms can be presented as discrete units, such as capsules, caplets, or tablets. In one embodiment, the invention provides a solid oral dosage form comprising a prodrug that is smaller in size compared to a solid oral dosage form containing a therapeutically equivalent amount of unbound drug. The smaller size of the prodrug dosage forms promotes ease of swallowing. For patients unable to swallow, a liquid formulation can be prepared for enteral administration via a feeding tube or for parenteral administration (e.g., injection).

Soft gel or soft gelatin capsules may be prepared, for example, by dispersing the formulation in an appropriate vehicle (e.g., vegetable oil) to form a high viscosity mixture. This mixture then is encapsulated with a gelatin based film. The industrial units so formed are then dried to a constant weight.

Chewable tablets can be prepared by mixing the prodrug with excipients designed to form a relatively soft, flavored tablet dosage form that is intended to be chewed. Conventional tablet machinery and procedures (e.g., direct compression, granulation, and slugging) can be utilized.

Film-coated tablets can be prepared by coating tablets using techniques such as rotating pan coating methods and air suspension methods to deposit a contiguous film layer on a tablet.

Compressed tablets can be prepared by mixing the prodrug with excipients that add binding qualities. The mixture can be directly compressed, or it can be granulated and then compressed.

The dose range of the prodrug will depend on a number of factors including the age, weight, and condition of the patient. Tablets and other dosage forms provided in discrete units can contain a daily dose, or an appropriate fraction thereof, of a prodrug. The dosage form can contain a dose of about 2.5 mg to about 500 mg, about 2.5 mg to about 250 mg, about 10 mg to about 100 mg, or increments therein of a prodrug.

The dosage form can utilize any one or any combination of known release profiles including, but not limited to immediate release, extended release, pulse release, variable release, controlled release, timed release, sustained release, delayed release, and long acting. Preferably, the prodrug releases the drug over a more extended period of time as compared to administering unbound drug.

The pharmaceutical compositions of the invention can be administered in a partial, i.e., fractional dose, one or more times during a 24 hour period. Fractional, single, double, or other multiple doses can be taken simultaneously or at different times during a 24 hour period. The doses can be uneven doses with regard to one another or with regard to the individual components at different administration times.

The dosage units can be packaged according to market need, for example, as unit doses, rolls, bulk bottles, blister packs, and so forth. The blister pack or other pharmaceutical package can optionally include or be accompanied by indicia allowing individuals to identify the identity of the pharmaceutical composition, the prescribed indication (e.g., major depressive disorder), and/or the time periods (e.g., time of day, day of the week, etc.) for administration. The blister pack or other pharmaceutical package can also include a second pharmaceutical product for combination therapy.

It will be appreciated that the pharmacological activity of the compositions of the invention can be demonstrated using standard pharmacological models that are known in the art. Furthermore, it will be appreciated that the inventive compositions can be incorporated or encapsulated in a suitable polymer matrix or membrane to enhance or achieve site-specific delivery, or can be functionalized with specific targeting agents capable of effecting site specific delivery. These techniques, as well as other drug delivery techniques, are well known in the art.

Any feature of the above-describe embodiments can be used in combination with any other feature of the above-described embodiments.

The invention is further illustrated by the following non-limiting examples.

### EXAMPLES

### Example 1. Preparation of amino acid succinate

To a solution of an N-protected amino acid (1.0 eq) in dioxane (22 ml/gram of amino acid), N-methylmorpholine (1.1 eq) and 1,3-dicyclohexylcarbodiimide (1.1 eq) were added. The solution was allowed to stir overnight at ambient temperature under argon. Then dicyclohexylurea was filtered off, and the filtrate was concentrated under reduced pressure. The product was recrystallized in acetone/hexane at 0°C and dried to afford the corresponding N-protected amino acid succinate.

### Example 2. Preparation of di- and tripeptide succinates

To a solution of amino acid (1.5 eq) in N,N-dimethylformarnide/dioxane/H₂O (2:2:1), N-methylmorpholine (3.0 eq) and N-protected amino acid succinate (1.0 eq) were added. The solution was allowed to stir overnight at ambient temperature under argon. Ethylacetate was then added, and the organic layer was washed with 2% acetic acid, water, brine and dried over sodium sulfate. The organic extract was concentrated and dried under vacuum to afford the dipeptide.

Dipeptide succinate can be prepared using the same process for preparing an amino acid succinate, described above.

Tripeptide succinates can be prepared by reacting an N-protected dipeptide succinate with an amino acid to form the tripeptide, and then converting the tripeptide to the succinate.

### Example 3. Preparation of tranylcypromine prodrugs

### a. Preparation of protected amino acid tranylcypromine

To a solution of protected amino acid succinate (1.0 eq) in ethyl acetate (7 ml) was added N-methylmorpholine (1.1 eq) and tranylcypromine ·½H₂SO₄ (1.5 eq). The solution was stirred overnight at ambient temperature under argon and then acidified by adding 1 N HCl. The organic layer was washed with sodium bicarbonate, dried over sodium sulfate, and concentrated. The crude product was then purified by column chromatography.

Di- and tripeptide conjugates of tranylcypromine can be prepared by using the di- or tripeptide succinate instead of the amino acid succinate.

### b. Deprotection

The protected amino acid tranylcypromine was dissolved in a solution of 4 N HCl in dioxane (15 ml) and allowed to stir overnight at ambient temperature under argon. The solvent was then removed under reduced pressure to afford the amino acid conjugate.

Exemplary peptides for tranylcypromine prodrugs include Ala, Arg, Asn, Asp, Gly, Glu, His, Lys, Met, Pro, Phe, Ser, Trp, Thr, Tyr, Phe₂, Gly₂, Glu₂, Pro₂, Lys₂, Asp₂, and Gly₃.

### Example 4. Preparation of fluoxetine and sertraline prodrugs

The prodrugs described in this example do not fall within the scope of the invention as claimed. However, the example has been retained as useful information to the reader.

The process for preparing fluoxetine and sertraline prodrugs is the same as the process for preparing tranylcypromine prodrugs, described above, except that tranylcypromine ·½H₂SO₄ is replaced with fluoxetine-HCl or sertraline-HCl. Exemplary peptides for fluoxetine prodrugs include Ala, Asp, Gly, Glu, lie, Leu, Lys, Pro, Phe, and Trp. Exemplary peptides for sertraline prodrugs include Boc-Phe and Boc-Gly.

The process can also be used to prepare prodrugs of other antidepressants having primary or secondary amino functionality, such as bupropion and fluvoxamine.

## Claims

1. A composition, or salt thereof, comprising tranylcypromine covalently bound to either
a single amino acid;
a dipeptide in which the two amino acids are the same; or
a tripeptide in which the three amino acids are the same and wherein the amino acid is a naturally occurring amino acid or a synthetic amino acid selected from the group comprising:
aminohexanoic acid, biphenylalanine, cyclohexylalanine, cyclohexylglycine, diethyglycine, dipropylglycine, 2,3-diaminoproprionic acid, homophenylalanine, homoserine, homotyrosine, naphthylalanine, norleucine, ornithine, phenylalanine (4-fluoro), phenylalanine (2,3,4,5,6-pentafluoro), phenylalanine (4-nitro), phenylglycine, pipecolic acid, sarcosine, tetrahydroisoquinoline-3-carboxcylic acid, tert-leucine, and an amino acid with a C₁-C₁₇ alkyl side chain.

2. The composition of claim 1, wherein the amino acid is an L-amino acid.

3. The composition of claim 1 or 2, wherein the trancylpromine is covalently bound to a single amino acid.

4. The composition of claim 2, wherein the tranylcypromine is covalently bound to Ala, Lys, Gly, Glu, Thr, Pro, Asp, Tyr, Met, Ser, Asn, Phe, His, Arg, Trp, Phe₂, Gly₂, Glu₂, Pyro₂, Lys₂, Asp₂, or Gly₃.

5. The composition of claims 1, wherein the composition is a hydrochloride salt or sulfate salts.

6. A composition as claimed in any of claims 1 to 5 for use in the treatment of a condition selected from the group consisting of major depressive disorder, obsessive-compulsive disorder, bulimia nervosa, panic disorder with and without agoraphobia, posttraumatic stress disorder, premenstrual dysphoric disorder, social anxiety disorder, major depressive episode without melancholia, generalized anxiety disorder, depression with and without prominent anxiety, endogenous depression, and nicotine withdrawal.

7. The composition of claim 6, wherein the condition is selected from the group consisting of major depressive disorder, obsessive-compulsive disorder, bulimia nervosa, panic disorder with and without agoraphobia, posttraumatic stress disorder, premenstrual dysphoric disorder, social anxiety disorder, and major depressive episode without melancholia.

8. The composition of claim 7, wherein the condition is major depressive disorder.

9. A pharmaceutical composition comprising a composition of any of claims 1 to 5 and at least one pharmaceutical additive.

## Patentansprüche

1. Zusammensetzung oder ein Salz dieser, mit einem Tranylcypromin, das kovalent gebunden ist entweder an
eine einzelne Aminosäure;
ein Dipeptid, bei dem es sich bei den beiden Aminosäuren um die gleichen Aminosäuren handelt; oder
ein Tripeptid, bei dem es sich bei den drei Aminosäuren um die gleichen Aminosäuren handelt, und wobei es sich bei der Aminosäure um eine natürlich vorkommende Aminosäure oder eine synthetische Aminosäure handelt, die aus der Gruppe ausgewählt wird, die folgendes umfasst:
Aminohexansäure, Biphenylalanin, Cycloheylalanin, Cyclohexyglycin, Diethylglycin, Dipropylglycin, 2,3-Diaminoproprionsäure, Homophenylalanin, Homoserin, Homotyrosin, Naphthylalanin, Norleucin, Ornithin, Phenylalanin (4-Fluor), Phenylalanin (2,3,4,5,6-Pentafluor), Phenylalanin (4-Nitro), Phenylglycin, Pipecolinsäure, Sarcosin, Tetrahydroisoquinolin-3-Carboxylsäure, Tert-Leucin und Aminosäure mit C₁-C₁₇ Alkylseitenkette.

2. Zusammensetzung nach Anspruch 1, wobei es sich bei der Aminosäure um eine L-Aminosäure handelt.

3. Zusammensetzung nach Anspruch 1 oder 2, wobei das Tranylcypromin kovalent an eine einzelne Aminosäure gebunden ist.

4. Zusammensetzung nach Anspruch 1, wboei das Tranylcypromin kovalent gebunden ist an Ala, Lys, Gly, Glu, Thr, Pro, Asp, Tyr, Met, Ser, Asn, Phe, His, Arg, Trp, Phe₂, Gly₂, Glu₂, Pro₂, Lys₂, Asp₂ oder Gly₃.

5. Zusammensetzung nach Anspruch 1, wobei es sich bei der Zusammensetzung um ein Hydrochloridsalz oder Sulfatsalz handelt.

6. Zusammensetzung nach einem der Ansprüche 1 bis 5 zum Einsatz in der Behandlung eines Zustands, der aus der Gruppe ausgewählt wird, die folgendes umfasst: ausgeprägte Depression, obsessive Zwangsstörung, Bulimia nervosa, Panikstörung mit und ohne Agoraphobie, posttraumatische Belastungsstörung, prämenstruelle dysphorische Störung, Sozialphobie, ausgeprägte depressive Verstimmung ohne Melancholie, generalisierte Angststörung, Depression mit und ohne Angststörung, endogene Depression und Nikotinentzug.

7. Zusammensetzung nach Anspruch 6, wobei der Zustand aus der Gruppe ausgewählt wird, die folgendes umfasst: ausgeprägte Depression, obsessive Zwangsstörung, Bulimia nervosa, Panikstörung mit und ohne Agoraphobie, posttraumatische Belastungsstörung, prämenstruelle dysphorische Störung, Sozialphobie und ausgeprägte depressive Verstimmung ohne Melancholie.

8. Zusammensetzung nach Anspruch 7, wobei es sich bei dem Zustand um eine ausgeprägte Depression handelt.

9. Pharmazeutische Zusammensetzung, die eine Zusammensetzung nach einem der Ansprüche 1 bis 5 und mindestens einen pharmazeutischen Zusatzstoff umfasst.

## Revendications

1. Composition ou sel de celle-ci comprenant de la tranylcypromine liée par covalence à :
un seul acide aminé ;
un dipeptide dans lequel les deux acides aminés sont identiques ; ou
un tripeptide dans lequel les trois acides aminés sont identiques et dans lequel l'acide aminé est un acide aminé se produisant naturellement ou un acide aminé synthétique sélectionné parmi le groupe comprenant :
l'acide aminohexanoïque, la biphénylalanine, la cyclohexylalanine, la cyclohexylglycine, la diéthylglycine, la dipropylglycine, l'acide 2,3-diaminopropionique, l'homophénylalanine, l'homosérine, l'homotyrosine, la naphtylalanine, la norleucine, l'omithine, la phénylalanine (4-fluoro), la phénylalanine (2,3,4,5,6-pentafluoro), la phénylalanine (4-nitro), la phénylglycine, l'acide pipécolique, la sarcosine, l'acide tétrahydroisoquinoline-3-carboxylique, la tert-leucine et un acide aminé avec une chaîne latérale d'alkyle en C₁-C₁₇.

2. Composition selon la revendication 1, dans laquelle l'acide aminé est un acide L-aminé.

3. Composition selon la revendication 1 ou 2, dans laquelle la tranylcypromine est liée par covalence à un seul acide aminé.

4. Composition selon la revendication 2, dans laquelle la tranylcypromine est liée par covalence à Ala, Lys, Gly, Glu, Thr, Pro, Asp, Tyr, Met, Ser, Asn, Phe, His, Arg, Trp, Phe₂, Gly₂, Glu₂, Pro₂, Lys₂, Asp₂ ou Gly₃.

5. Composition selon la revendication 1, dans laquelle la composition est un sel de chlorhydrate ou un sel de sulfate.

6. Composition selon l'une quelconque des revendications 1 à 5 destinée à être utilisée dans le traitement d'une affection sélectionnée parmi le groupe constitué d'un trouble dépressif majeur, d'un trouble obsessionnel-compulsif, de la boulimie, de la panique avec ou sans agoraphobie, d'un état de stress post-traumatique, d'un trouble dysphorique prémenstruel, d'un trouble d'anxiété sociale, d'un épisode dépressif majeur sans mélancolie, d'un trouble d'anxiété généralisée, d'une dépression avec ou sans anxiété proéminente, d'une dépression endogène et d'un sevrage tabagique.

7. Composition selon la revendication 6, dans laquelle l'affection est sélectionnée parmi le groupe constitué d'un trouble dépressif majeur, d'un trouble obsessionnel-compulsif, de la boulimie, de la panique avec ou sans agoraphobie, d'un état de stress post-traumatique, d'un trouble dysphorique prémenstruel, d'un trouble d'anxiété sociale et d'un épisode dépressif majeur sans mélancolie.

8. Composition selon la revendication 7, dans laquelle l'affection est un trouble dépressif majeur.

9. Composition pharmaceutique comprenant une composition selon l'une quelconque des revendications 1 à 5 et au moins un additif pharmaceutique.
